# EUROPEAN PATENT SPECIFICATION

(11) **EP 1 760 468 B1**
(45) Date of publication and mention of the grant of the patent: **14.10.2009**
(21) Application number: 05107912.7
(22) Date of filing: 30.08.2005
(51) Int. Cl.: G01N 33/68, G06F 19/00, G06F 17/00

(54) **Method for detecting a biochemical interaction**
Verfahren zum Nachweis einer biochemischen Interaktion
Méthode de détection d'une interaction biochimique

(43) Date of publication of application: 07.03.2007
(73) Proprietor: PerkinElmer Cellular Technologies Germany GmbH, 22525 Hamburg (DE)
(72) Inventor: Palo, Kaupo, Dr., 74001 Harju mk. (EE)
(74) Representative: von Kreisler Selting Werner

(56) References cited:
- WO-A-00/79268
- WO-A-03/081245
- WO-A-20/04109284
- WO-A-20/05029077
- KHALIFA MYRIAM BEN ET AL: "BIACORE data processing: An evaluation of the global fitting procedure" ANALYTICAL BIOCHEMISTRY, vol. 293, no. 2, 15 June 2001 (2001-06-15), pages 194-203, XP002357045

## Description

The invention relates to a method for detecting a biochemical interaction between at least two interaction partners.

For detecting the interaction of two biochemical partners, it is e.g. known to monitor the fluorescence change (counts) of various samples over the time. To generate a fluorescent signal, it is e.g. known to dispense as one interaction partner a potential agonist to cell-based samples, a receptor present in the cells being the second interaction partner. To perform a large number of tests, the interaction partners are e.g. dispensed into wells of a microtiter plate. These microtiter plates, having e.g. 96, 384 or 1536 wells which are commonly used to carry a variety of first interaction partners and identical second interaction partners, are analysed in high- or medium-throughput screening-devices. The reaction of the interaction partners and the presence of a fluorescent dye leads to a fluorescence signal for each well, whereby the intensity of the signal changes over time. To decide whether the interaction partners of one well are of interest, the maximum peak height (PH) of the fluorescence response signal or the area under the curve (AUC) of the response signal during a given time period is analysed. The problem of these parameters is that neither the area under the curve, nor the peak height of the response signal can distinguish between compound artefacts and relevant agonist responses. The artefacts can e.g. be caused by fluorescent compounds. In addition, it is not generally possible to distinguish agonists interacting specifically with the receptor under study from substances interacting unspecifically with a multitude of cellular receptors.

Therefore, in order to decide whether a potential agonist (hit) is of further interest or not, it is necessary according to the state of the art to perform a confirmation screen, typically using identical concentrations and conditions of the first interaction partners. Subsequent steps frequently include a selectivity screen, using e.g. the interaction with a different receptor or a receptor isoform, and/or a parental screen with a receptor negative cell line for comparison. Also, a validation screen using different concentrations of the first interaction partner may be performed to establish dose response relationships. During each step of this method the hits are further characterized. The goal here is to remove those hits that have undesirable properties and to concentrate on those hits which pass the selection criteria. It is quite common that at one or more steps during this method chemists select a subset of hits that will be taken to the next step based on the presumed likelihood that a hit can be converted into a molecule with drug like properties. The entire method is e.g. described in: A.D. Baxter and P.M. Lockley, "'Hit' to 'lead' and 'lead' to 'candidate' optimisation using multi-parametric principles", Drug Disc. World Winter 2001/2.

Analysing methods dealing with the study of binding kinetics are known from WO 2005/029077, WO 2004/109284 and WO 2003/081245.

Since it is necessary to perform these additional screens, the known method is expensive and time-consuming. It is an object of the invention to improve the method for detecting a biochemical interaction, whereby the analysis of the derived data shall be improved leading to a better prediction of chemically attractive compounds for follow-up studies. This and other objects are solved by a method according to claim 1.

According to the invention, the method for detecting a biochemical interaction between at least two interaction partners comprises the following steps:
- bringing into contact the at least two interaction partners ,
- taking a temporal sequence of measurements, each of them producing a measurement value describing the state of the interaction at a given point in time,
- choosing a mathematical model to describe the temporal sequence of measurements, whereby the model comprises at least one first parameter characterising a temporal phase of increasing measurement values and at least one second parameter characterising a temporal phase of decreasing measurement values,
- adapting the mathematical model to the temporal sequence of measurements, whereby values for said parameters are determined which
- result in a good approximation of the temporal sequence of measurements by the mathematical model, and
- detecting the biochemical interaction by evaluating the first and second parameter and/or a measure of deviation of the mathematical model from the temporal sequence of measurements,
   the interaction between at least two interaction partner results in a change of a fluorescent signal from a fluorescent reporter, where the fluorescent report is a potential sensitive dye, an ion sensitive dye or pH sensitive dye, whereby at least one interaction partner is a biochemical receptor, an ion channel or an ion pore, and at least one interaction partner is located in or on a cell, vesicle, organic tissue.

Since according to the invention a mathematical model, e.g. a mathematical describable curve, is adapted to the temporal sequence of measurements, a first and second parameter can be determined. These parameters can be used to decide whether the substances within a well are of interest or not. Within a preferred embodiment using specific relevant parameters of the mathematical model, it may not be necessary to perform an additional confirmation, parental, selectivity and/or validation screen. Thus, the information of interest can be derived quicker. Additionally, the screening costs can be decreased further, by saving costs for labour chemical compounds and reagents required to carry out those secondary screens.

The interaction partners that are brought together in the first step, are e.g., small organic molecules (chemical compounds), proteins, peptides, polynucleotide strands, natural cellular receptors or target receptors of interest expressed heterologously in a cell line. The performed measurement is e.g. the measurement of the fluorescence intensity over the time. To provide for a fluorescent read-out, typically a fluorescent dye is also added to the sample comprising the two interaction partners. Depending on the biochemical reaction to be studied., this may be an ion sensitive dye (e.g. a calcium sensitive dye), a potential sensitive dye or a pH sensitive dye. Specifically, dyes of the bis-barbituric acid oxonol type may be used such as Dibac₄(3), DiSBAC₂(3) or DiBAC₄(5) which are commercially available (e.g. by the supplier Molecular Probes). Also other oxonol dyes such as bis-isoxazolone oxonol dyes (e. g. Oxonol V and Oxonol VI) may be applied. Further voltage-sensitive indicators include carbocyanine derivatives (e.g. indo-, thia-, and oxa-carbocyanines as well as iodide derivatives of carbocyanines), rhodamine dyes, merocyanine 540 and styryl dyes. Among the styryl dyes, one might apply dyes of the aminonaphtylethenylpyridinium type such as di-4-ANEPPS, di-8-ANEPPS, di-2-ANTPEQ, di-8-ANEPPQ, di-12-ANFPPQ or di-1-ANLPIA which are all commercially available(Molecular Probes). Also RH-dyes of this or other suppliers may be used such as RH 414, RH 421, RH 795 or RH 237. As ion-sensitive indicators one might use well-known and commercially available calcium indicators (e.g. fluo-calcium indicators, fura indicators, indo indicators, Calcium Green^{™} or Oregon Green^{™}; Molecular Probes) or sodium/potassium indicators (e.g. SBFI, PBFI, Sodium Green Na⁺ indicator, CoroNa Green Na⁺ indicator, CoroNa Red Na⁺ indicator; Molecular Probes).

The mathematical model or curve which is according to the invention preferably fitted to a part of the curve derived by the measurements, may be a straight line. In general, the curve derived by the measurements, has an increasing and a decreasing part or temporal sequence. The curve may either comprise an essentially increasing section, followed in time by an essentially decreasing section, or vice versa. Thus, in a first embodiment, two straight lines can be fitted to the curve of the measurements.

Preferably, separate functions are used to describe the rise and decay of the sequence of measurements, e.g. to describe the increasing section and the decreasing section of the curve derived by the measurements. In a preferred embodiment, the mathematical models or curves fitted to the two sections of the measurement, curve are segments of Gauss-functions, especially preferred half Gauss-functions.. Either single Gaussian functions, or a superposition (i.e. a linear combination) of multiple segments of Gaussian functions can be used to fit each segment. Particularly, a superposition of two Gauss-curve segments is fitted to the decreasing section of the curve in one embodiment. It is also possible to divide the measured curve into more than two portions, whereby in each portion a mathematical model or curve is fitted to the measurement curve.

An additional advantage of the method according to the invention is that it is possible to store the parameters describing the mathematical models or curves instead of the raw measured data, whereby the data volume compared with the raw data is reduced. Particularly, the data volume is less than 20%, particularly less than 10% of the raw data.

The parameters of interest of the measurement curve and/ or the fitted curve are the width (i.e. rise time) of the ascending curve, the width (i.e. decay time) of the descending curve, width of the descending curve squared as well as the Chi-squared, which is a measure for the consistency between measured curve and fitted curve known in the art. If more than two curves are fitted to the measurement curve, the width of each curve is of interest. In addition, the maximum and minimum height, the area under the curve, the mean height, the standard deviation of height, the amplitude and the position in time of the maximum can be obtained.

The mathematical models may be adapted via numerical least-squares fit. This approach, which is well known in the art, aims to minimise the mean square deviation between the measured data points and the mathematical model. Algorithms for carrying out the least squares fit for linear or non-linear mathematical models (e.g. Levenberg-Marquardt method) are known in the art (see e.g. W.H. Press at al., Numerical Recipes in C, Cambridge University Press, Cambridge 1992)

An important advantage of the method according to the invention is that within the last step, the detecting of the biochemical interaction by evaluating the first and second parameter, specific and non-specific interaction can be discriminated. Additionally, it is possible to discriminate between valid sequences of measurements and sequences of measurements influenced by measurements artefacts. The measurement artefacts may comprise auto fluorescence, and cytotoxic compounds.

According to a preferred embodiment of the invention, at least one interaction partner is a biochemical receptor. Preferably, one interaction partner is located in or on a cell, receptor, organic tissue, carrier particle consisting of organic or inorganic matter, carrier surface consisting of organic or inorganic matter or the like. The second interaction partner is preferably a chemical compound. One or both of the interaction partners may be dissolved or suspended in a liquid media or assay buffer.

Preferably, the interaction of a first interaction partner (e.g. a receptor) with a second interaction partner (e.g. a chemical compound) is investigated in high throughput experiments, and statistical analysis of the resulting multitude of model parameters is used in the detecting step.

A preferred embodiment of the method according to the invention will be described in detail on the basis of the enclosed figures:
- Fig. 1: shows schematically two of the early steps of a method according to the state of the art.
- Fig. 2: is a diagram of the peak height of fluorescence intensity traces obtained in a confirmation screen over the peak height of fluorescence intensity traces obtained in a selectivity screen.
- Fig. 3: shows raw data as well as fit results of responses elicited by a positive control (left panel), a chemically attractive compound (centre panel) and an autofluorescent compound (right panel), respectively
- Fig. 4: shows χ² values, quantifying the fit between measured data and mathematical models, for data obtained in a confirmation and a parental assay.
- Fig 5: shows χ² values, quantifying the fit between measured data and mathematical models, for data obtained in a confirmation assay. Shown are χ² values for all compound as well as those compounds that have been selected for the validation screen (IC₅₀ determination) and examples from the eight compound series.
- Fig. 6: is a diagram of the normalized width of the ascending curve over the normalized width of the descending curve of fluorescence intensity traces obtained from all compounds tested in the confirmation screen. In addition, five representative example fluorescence intensity traces are shown.
- Fig. 7: presents the same diagram as Fig. 6, showing only a subset of compounds. Blue labelled compounds are those that have been inactive in the parental screen and are therefore considered selective. Red labelled compounds are those that have also been active in the parental screen and are therefore considered non-selective. The green rectangle encompasses those compounds with kinetics that are within ~ 10 fold of the mean normalized response
- Fig. 8: shows the central section of Fig. 7 at higher resolution. The outermost green rectangle encompasses those compounds with kinetics that arc within ~ 10 fold of the mean normalized response. The other green rectangles encompass those compounds with kinetics that arc within ~ 5 fold, 2 fold. 1..5 fold and 1.2 fold of the mean normalized response, respectively..
- Fig. 9: shows the number of compounds interacting selectively and non-selectively, as a function of the selected range of rise and decay times of their fluorescence intensity over time.

### Example

In this example, the method of the invention was used to investigate the interaction of various different chemical compounds with a G-protein coupled receptor (GPCR) heterologously expressed in a mammalian HEK-293 cell line It was the aim of this study to identify specific agonists for the GPCR, i.e. compounds which interact specifically with the receptor under study by binding to the receptor and triggering an intracellular response via a second messenger system, but neither interact with other receptors present in the same cell line nor trigger a comparable response by another mechanism.
7680 assay wells were used in this example analysis of which 5238 were compounds, 480 controls and 1962 wells to which only a solvent (DMSO) without compound was added.
The initial screen identified 5338 compounds of further interest of which 500 were selected based on chemistry triage.

As is well known to those skilled in the art, binding of extracellular compounds to GPCRs causes an intracellular signal cascade, mediated by G proteins coupling to the receptor. Specifically, agonists acting on the GPCR under study will in a first phase trigger an increase in the intracellular level of Ca²⁺ ions. In a second phase, - through various mechanisms, including re-entry of Ca²⁺ ions in the endoplasmatic reticulum (ER) - the Ca²⁺ level in the cells under study decreases again.

It is well-known in the art to observe the intracellular level of Ca²⁺ ions via the use of fluorescent "calcium indicator" dyes. These dyes exhibit shifts in fluorescence excitation and/or emission spectra, and/or emission levels, upon binding to Ca²⁺. By introducing these dyes into the cytoplasm, and then observing fluorescence emission in a series of measurements before and during exposure of the cells to a potential agonist, the change of intracellular Ca²⁺ levels can be followed over time as a change in observed fluorescence intensity and/or wavelength.

In this study, the "Fluorescent Imaging Plate Reader" (FLIPR) as well as the No Wash Calcium Indicator Dye (Molecular Devices #R8033), both commercially available from Molecular Devices, Inc., were used to measure fluorescent intensities. In preparation for the measurements non-adherent HEK-293 cells and compounds were pre-dispensed into separate plates prior to start of assay. The screen was performed in the 384 well plate format with 360 compounds and 24 controls per plate. Compounds were transferred to the wells containing cells using the integrated pipetting device of the FLIPR. The temporal evolution of fluorescence from the Calcium Indicator Dye, while irradiated with 488 nm excitation light, was then observed and recorded using the FLIPR reader. Time scales, addition speeds and dispense heights were pre-defined by the user in the FLIPR software.

In the prior art, the FLIPR data are typically evaluated by determining the peak height (maximum measurement value,) and / or the integrated fluorescence (area under the curve,) of the fluorescence signal. It is known in the art that no information about the specificity of the agonist's interaction with the receptor can be gained from this evaluation.. Large values of integrated fluorescence and/or peak height may indicate the presence of a specific agonist. However they may also be due to an unspecific interaction either with another receptor or an entirely different mechanism which is undesirable, or due to measurement artefacts (which may indicate activity for a non-active compound). The latter notably include the presence of autofluorescent compounds, which may falsify the observed fluorescence signals. It is therefore customary in the prior art to follow the primary screen with secondary screens aiming at eliminating unspecific binders as well as invalid agonists associated with measurement artefacts.

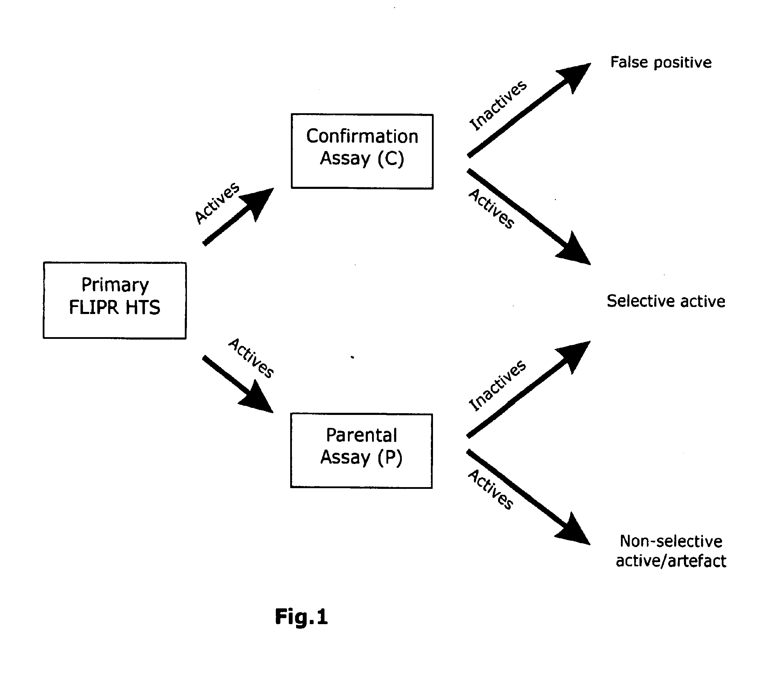

Fig. 1 shows two of the early steps of this method: All apparently active compounds are re-screened in a confirmation screen and a so-called parental screen. In the confirmation screen those compounds that have shown activity in the primary screen (bits) are re-screened under identical conditions and concentrations on the same platform. With the confirmation screen it can thus be confirmed that the hits truly show activity. In the parental screen the interaction of the hits with a cell line not exhibiting the receptor under study (a "receptor negative cell line") is measured to exclude that a hit unspecifically interacts with multiple receptors. A response in a. receptor negative cell line indicates the compound is acting at an alternative receptor or is fluorescent.

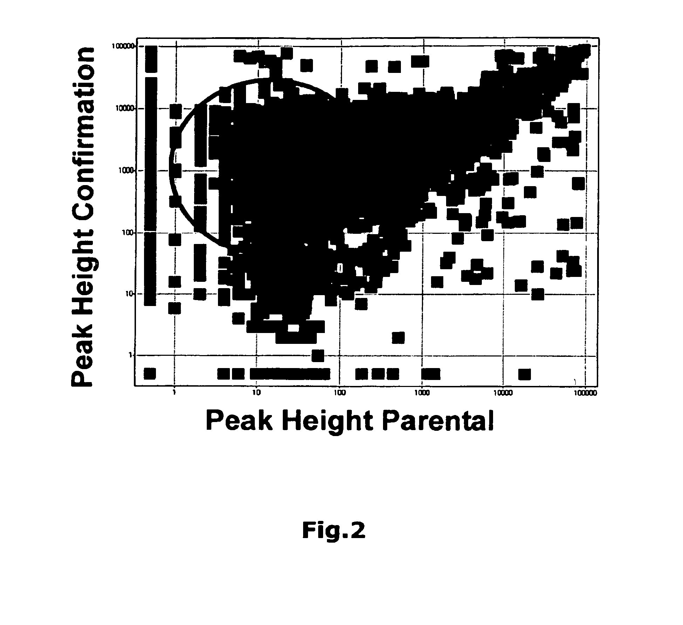

Fig 2 illustrates the limitations of the prior art approach. In particular, it confirms that no information about specificity of interactions is available from the evaluation parameters used in the art, i.e. peak height and area under curve. Fig 2 shows the peak heights as derived from the confirmation and parental screen. As apparent from Fig. 2, for many compounds there is a strong correlation between peak heights observed in the confirmation and parental screen - this means that most compounds observed as highly active in the confirmation (and hence also the primary) screen are actually interacting with the receptor non-specifically, and are hence of no pharmacological use. However, a substantial fraction of compounds - encompassed by the black circle show substantial activity in the confirmation and little activity in the parental screen, indicating that they are selective. Unfortunately, when following the approach known in the art, the costly confirmation and parental screen needs to be carried out for this large number of compounds before one can distinguish between these two populations.

The confirmation and parental screen as well as the other additional validation steps collectively referred to as secondary screens involve high labour costs as well as additional consumption of potentially expensive compounds and reagents. The method of the present invention therefore aims at identifying specific agonists without costly secondary screens.

Nevertheless, the confirmation and parental screens are carried out in this example to demonstrate and verify the benefits of the invention.

According to the invention, new evaluation parameters can be derived from the observed time sequence of fluorescence intensity data, which allow to predict the specificity of agonist interaction directly from the primary screen's results. To this end, a mathematical model which contains one first parameter characterising a temporal phase of increasing fluorescence intensity values and at least one second parameter characterising a temporal phase of decreasing fluorescence intensity values is fitted to each temporal sequence of fluorescence intensity values. Specifically, the model chosen here fits the temporal sequence in two separate segments: A single Gaussian function, ${f}_{0} \left(t\right) = {a}_{0} * exp \left[{{s}_{0}}^{2}/{\left(t-{t}_{0}\right)}^{2}\right] | t < {t}_{0}$
is fitted to the segment showing increasing fluorescence intensity values, and a superposition of two Gaussian functions, ${f}_{1} \left(t\right) = {a}_{1} * exp \left[{{s}_{1}}^{2}/{\left(t-{t}_{0}\right)}^{2}\right] + \left({a}_{0}-{a}_{1}\right) * exp \left[{{s}_{2}}^{2}/{\left(t-{t}_{0}\right)}^{2}\right] | t > {t}_{0}$
is fitted to the segment showing decreasing fluorescence intensity values. Here, t₀ denotes the time when the maximum fluorescence intensity is observed, a₀ denotes the maximum fluorescence intensity value, and s₀, s₁ and s₂ denote typical rise and decay times, respectively. By convention, s₁ is used to denote the faster of the two decay components, i.e. s₁ < s₂ An additive term accounting for basal fluorescence has been omitted for clarity.

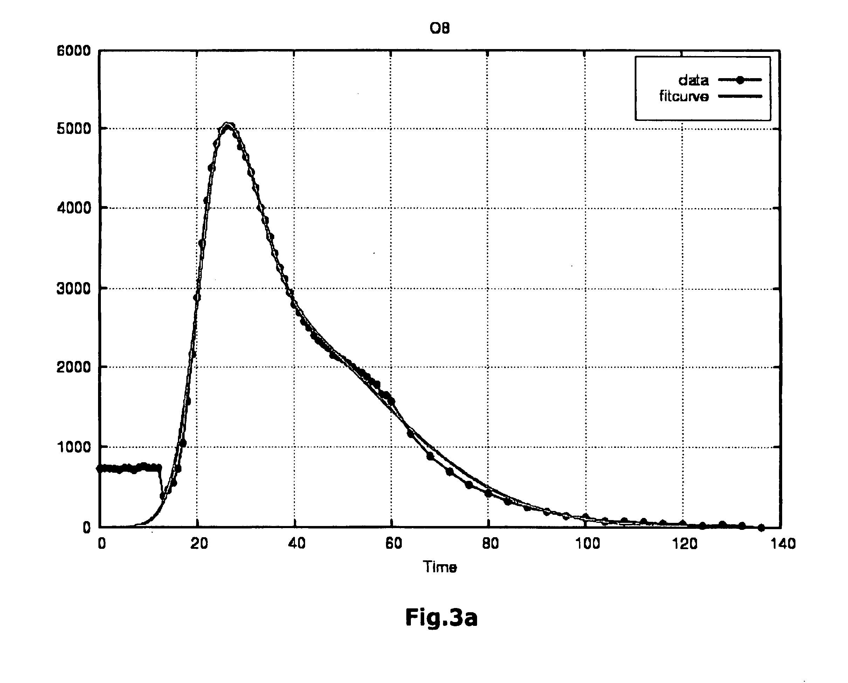

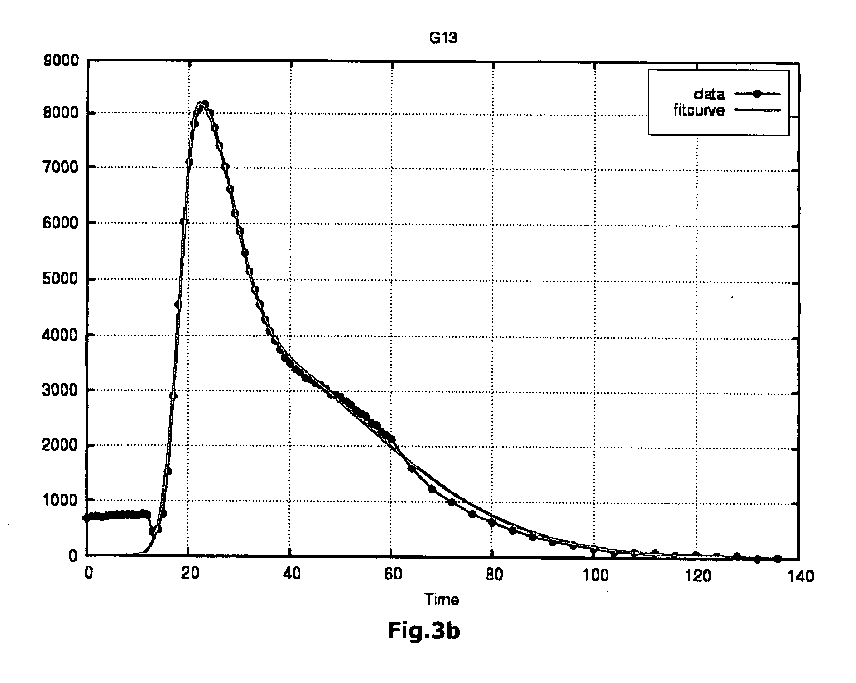

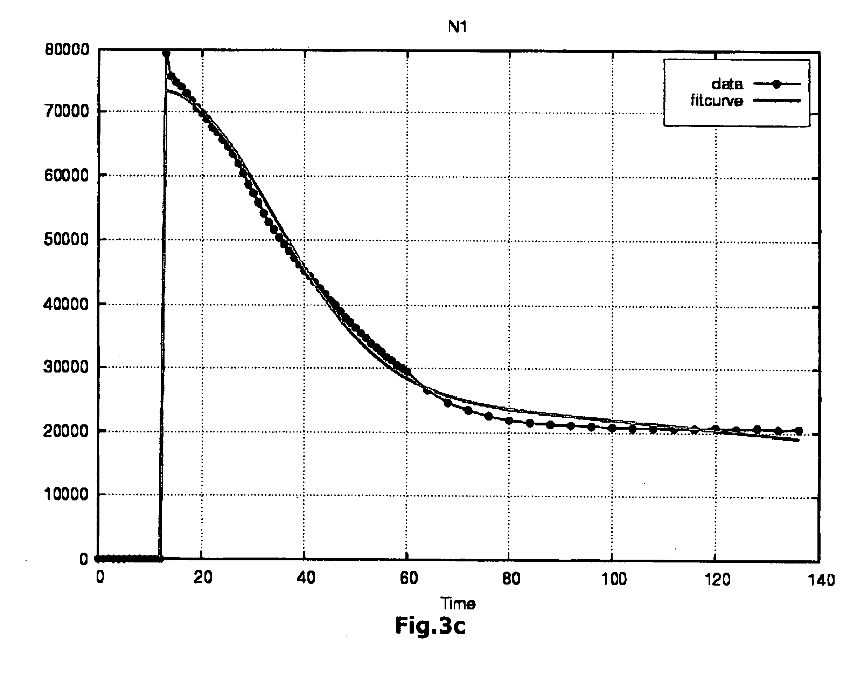

Fig 3 shows three example flourescent intensity traces that have been elicited by three different compounds (Color 1 line) Superimposed arc fit results (Color2 line) where the fluorescent signals have been fitted according to the model outlined above. The first example (left panel) shows the response to the maximum control compound. A compound that after a series of validation screens has eventually been selected as a compound attractive for medicinal chemists has induced the second flourescent intensity traces (middle panel). The third response (right panel) stems from a compound known to give rise to a false positive hit because it is autofluorescent. These three examples serve to illustrate two elements that are central to the method of the invention i) the mathematical model fits the rise and decay times of the compound elicited responses and ii) the quality of how well the model describes the raw data - which can mathematically be described with a χ² (Chi2) value - differs between different compounds. The benefits of using these elements for data analysis is outlined below.

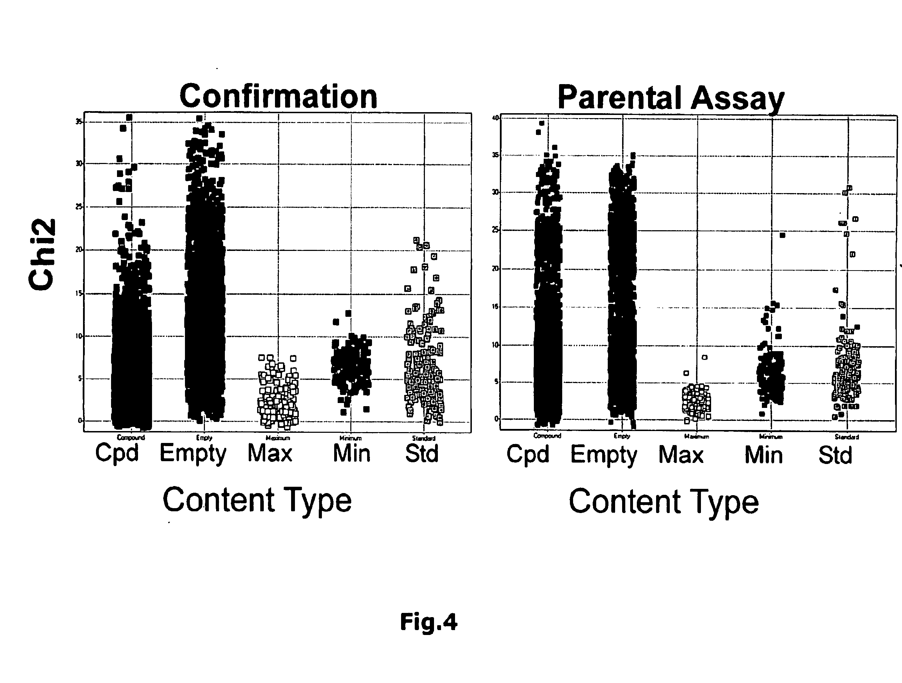

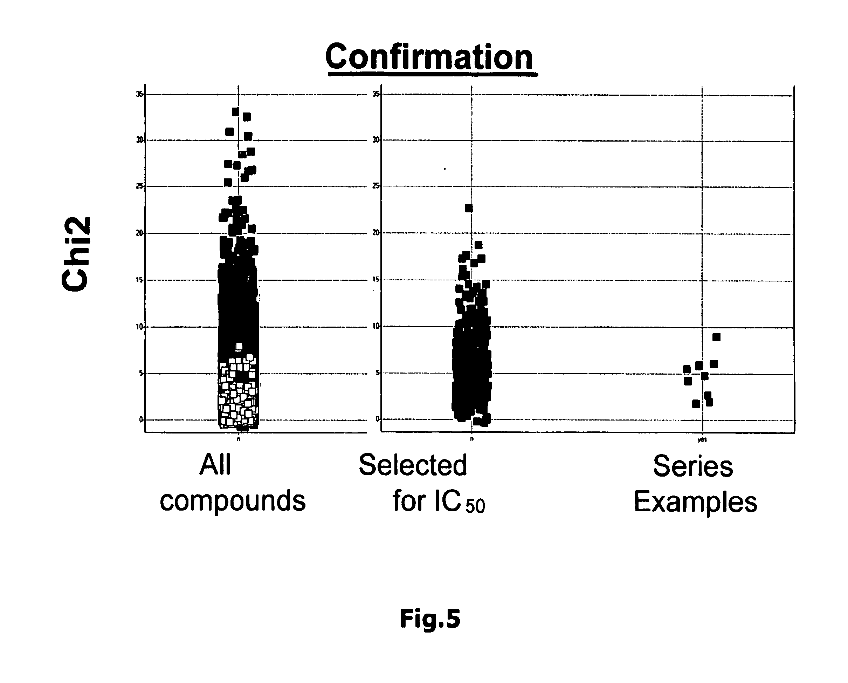

Although the observed data sets stem from complex cellular responses, they are fit quite well by the functional model. Fig. 4 shows χ² (Chi2) values for all compounds under investigation, as well as for a number of control samples, i.e. substances known to induce in the cells a minimal response, a maximal response and a "standard" response (defined here as 50% of the maximum response). The standard definition for the χ² value in mathematical statistics is used, i.e. essentially a normalized mean square deviation between functional model and actual measurement sequences. In the present statistical ensemble, a χ² value of less than 5, as observed for all maximum and standard controls, indicates very good agreement between functional model and measurement sequences. As expected, higher χ² values are observed for the minimum control samples, which exhibit generally weak fluorescence signals, and for some of the actual compounds under investigation, which are associated with artefacts including autofluorescence. It is worth pointing out that for the compounds (shown in red) there are more fits with low χ² values for the confirmation screen than for the parental screen. Since the costs of the various secondary screens are considerable it is quite common not to advance all compounds that have shown to be selective from one screen to the next. Instead a fraction of the selective compounds are chosen according to a variety of criteria including but not limited to physicochemical properties that are thought to be indicative of the likelihood that a compound can be modified to become a drug. In this endeavour it is very helpful in case compound series with similar structure but variable side chains can be identified that prove to be selective. Fig. 5 shows χ² values for all compounds as derived from the fits to the responses they elicited in the confirmation screen. The middle and right column show the χ² values of those compounds that were later selected for a validation screen (IC50 determination) and example compounds from eight different compound series. This illustrates that the behaviour of those compounds that prove chemically attractive are described and fit very well by the mathematical model. In other words, based on the quality of the fit (as indicated by a low χ²) of the model to primary screen data one can select a pool of compounds that are attractive for further optimization by medicinal chemists thus avoiding some of the time and cost intensive secondary screens.

Based on the described mathematical model it is possible to describe all or parts of the temporal sequence with a different kinds of parameters such as for example the rise and decay times. Two such parameters that have been used in the present study presented include the 'Normalised Width Ascending' and the 'Normalised Width Descending' which in this particular case have been calculated as follows. The median value of all fit results were calculated for the 12 High Control wells on each plate. The normalised fit results for a well were defined as the response of the well divided by the median response of the corresponding High Control wells on the same plate. Therefore, the 'Normalised Width Ascending' of a well is the width ascending fit result for that well, divided by the median width ascending of the High Control wells on that plate. Similarly, 'Normalised Width Descending' of a well is the width descending fit result for that well, divided by the median width descending of the High Control wells on that plate.

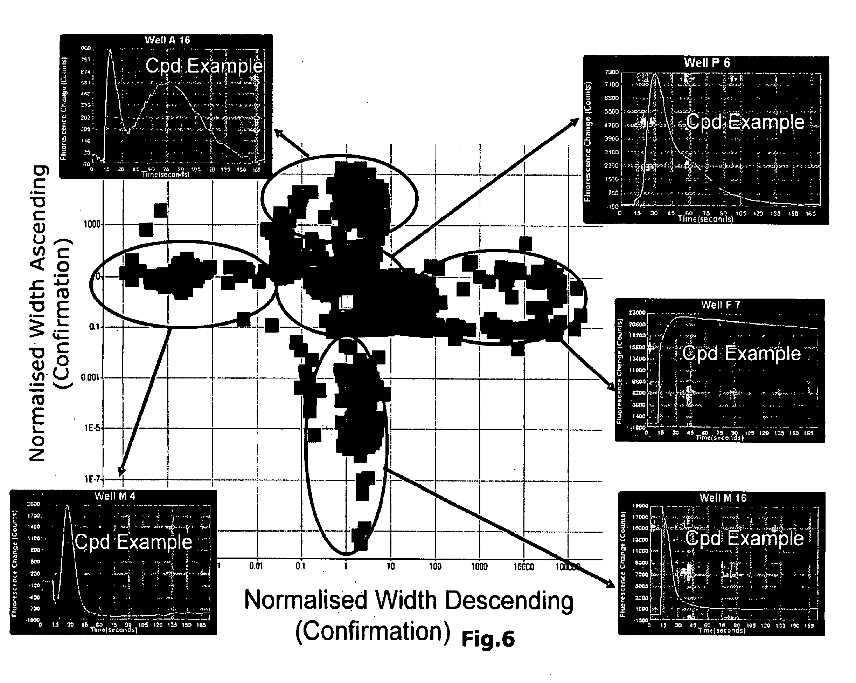

Following the method of the invention, we now investigate The fit parameters Normalized Width Ascending (NWA) and Normalized Width Descending (NWD) determined above. Fig. 6 illustrates the wide distribution of NWA and NWD for the compounds under investigation. The upper right inset shows an example of a compound inducing a response with rise and decay kinetics very similar to the maximum controls (shown in yellow). The other insets provide examples of a compounds inducing various combinations of similar, shortened and prolonged rise and decay times. Most notably, extended decay times and shortened rise times are often observed. Since these extended or shortened times are not observed in the control samples which are known to be specific agonists, it can be hypothesized that they are due to measurement artefacts and/or unspecific interactions exhibiting different kinetic behaviour. Following this hypothesis, we select only those compounds under investigation associated with fluorescence rise and decay times in the range observed for the control samples (indicated by the central black ellipse in Fig. 6).
With the next three figures we show how two fit parameters of the method of the invention (NWA and NWD) can be used to chose selective compounds from the primary screen in a quantitative manner. This is illustrated by using data from the confirmation and parental screens. In the method used in the state of the art these secondary screens arc performed tp allow one to distinguish between selective and non-selective compounds.

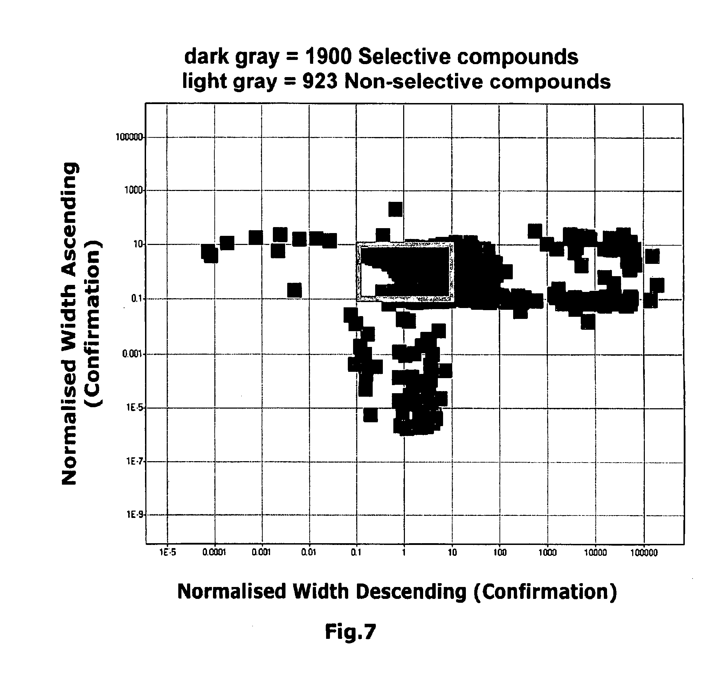

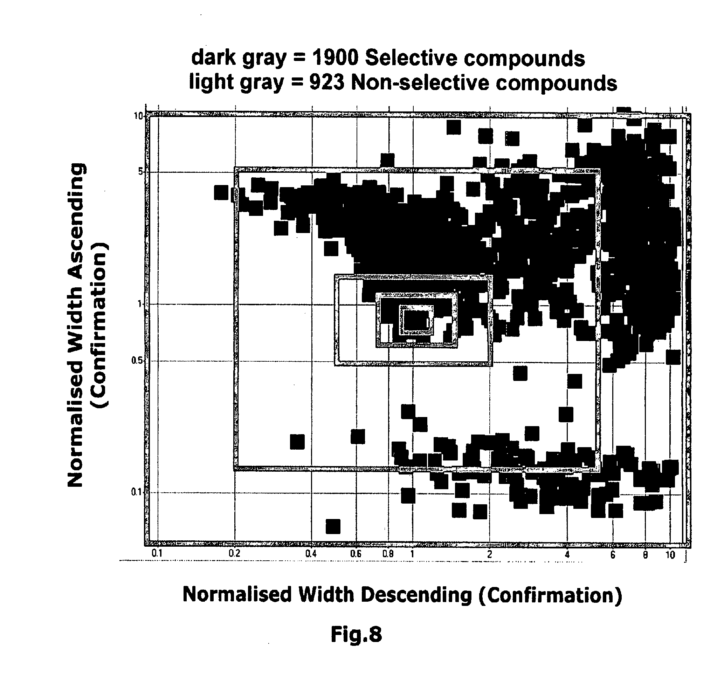

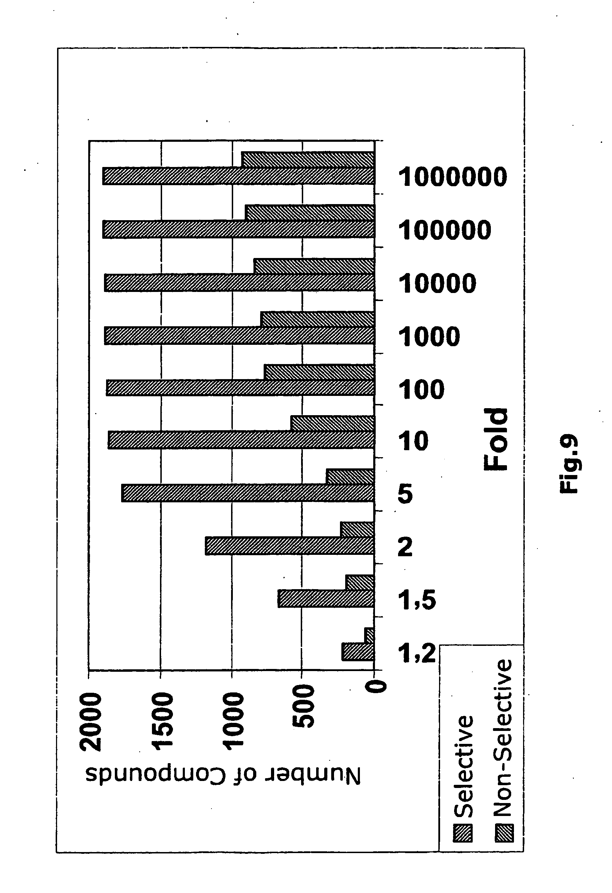

Fig. 7 shows the NWA plotted against the NWD obtained from fits to compound induced responses of the confirmation screen. Depicted in blue are those compounds that have shown to be active in the confirmation and non-active in the parental screen. They are thus classified as selective. The red labelled compounds are the non-selective ones since they have shown activity in the confirmation and the parental screen. Interestingly, 1880 out of the 1900 (98,9%) of the selective compounds have kinetics within 10-fold of the normalized response shown by the maximum control that is illustrated by the green rectangle. The fraction of non-selective compounds within in this area is much lower. This is better illustrated in Fig. 8, which shows the same plot as Fig.. 7 at higher magnification. The outermost green rectangle encompasses the same area as the green rectangle in Fig. 7. The inner green rectangles encompass those compounds with kinetics that are within ∼ 5 fold, 2 fold 1.5 fold and 1.2 fold of the mean normalized response of the maximum control, respectively. By closing in onto the mean response exhibited by the maximum controls, one gets the impression that the ratio of selective over non-selective compounds increases. This has been further quantified in Fig. 9 which shows the number of selective (red) and non-selective (blue) compounds within the areas that are 1.2 to 1000000 fold of the normalized response shown by the maximum control. In this example the biggest enrichment for selective over non-selective compounds (5.4 to 1) takes place for responses that arc within five fold of the standard response.
In summary, figs. 7 to 9 illustrate one example of a strategy to enrich selective over non-selective compounds at a very early screening stage by using both the kinetics of the rising and the decay phase. However one could also think of alternative strategies. One such strategy could for example be to look for compounds that have an up to five fold increased NWA and a NWD ranging from five fold decreased to two fold increased. All such strategies have in common that fitting the rise and decay times of a compound elicited response enables the comparison to kinetics displayed for the maximum control compound and the prediction of whether the compound under investigation is likely to be selective or non-selective. In summary this example therefore shows that, by characterising the interaction between a compound and a receptor according to the rise and decay times of the observed time sequence of measured fluorescence intensities, valuable information towards the validity and specificity of the interaction can be gained, without the need for costly secondary screens.

## Claims

1. A method for detecting a biochemical interaction between at least two interaction partners, comprising the steps of
- bringing into contact the at least two interaction partners ,
- taking a temporal sequence of measurements, each of them producing a measurement value describing the state of the interaction at a given point in time,
- choosing a mathematical model to describe the temporal sequence of measurements, whereby the model comprises at least one first parameter characterising a temporal phase of increasing measurement values and at least one second parameter characterising a temporal phase of decreasing measurement values,
- adapting the mathematical model to the temporal sequence of measurements, whereby values for said parameters are determined which result in a good approximation of the temporal sequence of measurements by the mathematical model, and
- detecting the biochemical interaction by evaluating the values of the first and second parameter and/or a measure of deviation of the mathematical model from the temporal sequence of measurements, n the interaction between the at least two interaction partners results in a change of a fluorescent signal from a fluorescent reporter, where the fluorescent reporter is a potential sensitive dye, an ion sensitive dye or a pH sensitive dye,
whereby
at least one interaction partner is a biochemical receptor, an ion channel or an ion pore, and
at least one interaction partner is located in or on a cell, vesicle, organic tissue.

2. Method according to claim 1, whereby the temporal sequence of measurement values comprises a temporal phase of increasing measurement values and/or a temporal phase of decreasing measurement values.

3. Method according to claim 1 or 2, whereby the at least one first parameter characterises a rise time or rise rate corresponding to the temporal phase of increasing measurement values, and whereby the at least one second parameter characterises a decay time or decay rate corresponding to the temporal phase of decreasing measurement values.

4. Method according to one of the claims 1 - 3, whereby the mathematical model uses separate functions to describe the phases of increasing and decreasing measurement values of the sequence of measurements.

5. Method according to one of the claims 1 - 4, whereby two mathematical models are used to describe the phase of decreasing measurement values of the sequence of measurements.

6. Method according to one of the claims 1 - 5, whereby the mathematical model uses sections of single Gaussian functions or a superposition of sections of multiple Gaussian functions to describe the phase of increasing measurement values and the phase of decreasing measurement values.

7. Method according to one of the claims 1 - 6, whereby the mathematical model is adapted via numerical least squares fit.

8. Method according to one of the claims 1 - 7, whereby at least one interaction partner is a G-protein Coupled Receptor.

9. Method according to one of the claims 1 - 8, whereby at least one interaction partner is dissolved or suspended in a liquid.

10. Method according to one of the claims 1 - 9 whereby the interaction of a first interaction partner with a multitude of second interaction partners is investigated in a multitude of experiments, each experiment comprising the steps of
- bringing into contact the at least two interaction partners ,
- taking a temporal sequence of measurements, each of them producing a measurement value describing the state of the interaction at a given point in time,
- choosing a mathematical model to describe the temporal sequence of measurements, whereby the model comprises at least one first parameter characterising a temporal phase of increasing measurement values and at least one second parameter characterising a temporal phase of decreasing measurement values,
- adapting the mathematical model to the temporal sequence of measurements, whereby values for said parameters are determined which result in a good approximation of the temporal sequence of measurements by the mathematical model,
and thereafter using statistical analysis of the resulting multitude of values of said parameters and/or measures of deviation of the mathematical model from the temporal sequence of measurements in detecting the multitude of biochemical interactions.

11. Method according to one of the claims 1 - 10, whereby a starting point of a temporal evolution of the biochemical interaction is defined by bringing the interaction partners into contact.

12. Method according to one of the claims 1 - 11, whereby a starting point of a temporal evolution of the biochemical interaction is defined by a first external triggering event, after the interaction partners have been brought into contact.

13. Method according to one of the claims 1 - 12, whereby a change in a direction of a temporal evolution of measurement values is defined by a second external triggering event, wherein said change comprises a transition from a phase of increasing to a phase of decreasing measurement values, or vice versa.

14. Method according to one of the claims 1 - 13, whereby luminescence signals, preferably fluorescent signals, are measured to produce measurement values describing the state of the interaction.

15. Method according to one of the claims 1 - 14, whereby the step of detecting the biochemical interaction by evaluating the values of the first and second parameter and/or the measure of deviation of the mathematical model from the temporal sequence of measurements provides information on the specificity of the interaction.

16. Method according to one of the claims 1 - 15, whereby the step of detecting the biochemical interaction by evaluating the values of the first and second parameter provides information on the effect of measurement artefacts on the measurement values.

17. Method according to one of the claims 1 - 16, wherein determining the measure of deviation of the mathematical model from the temporal sequence of measurements comprises the following steps:
- for a multitude of measurement values selected from the temporal sequence of measurements, calculating the difference between each measurement value and the corresponding value of the mathematical model, wherein the at least one first and second parameter determined by adapting said model to said sequence of measurements are used in the model,
- calculating the squares of said differences,
- calculating a weighted sum of said squares, wherein the weight for each square preferably depends on the corresponding measurement value or value of the mathematical model.

## Patentansprüche

1. Verfahren zum Erkennen einer biochemischen Interaktion zwischen mindestens zwei Interaktionspartnern, mit den folgenden Schritten:
- Bringen der mindestens zwei Interaktionspartner in Kontakt miteinander,
- Durchführen einer zeitlichen Abfolge von Messungen, die jeweils einen Messwert erzeugen, welcher den Zustand der Interaktion zu einem bestimmten Zeitpunkt beschreibt,
- Wählen eines mathematischen Modells zur Beschreibung der zeitlichen Abfolge von Messungen, wobei das Modell mindestens einen ersten Parameter, welcher eine Zeitphase ansteigender Messwerte kennzeichnet, und mindestens einen zweiten Parameter aufweist, welcher eine Zeitphase abnehmender Messwerte kennzeichnet,
- Anpassen des mathematischen Modells an die zeitliche Abfolge von Messungen, wobei Werte für die Parameter bestimmt werden, welche zu einer guten Annäherung an die zeitliche Abfolge der Messungen durch das mathematische Modell führen, und
- Erkennen der biochemischen Interaktion durch Auswerten der Werte des ersten und des zweiten Parameters und/oder einer Messung der Abweichung des mathematischen Modells von der zeitlichen Abfolge der Messungen, wobei die Interaktion zwischen den mindestens zwei Interaktionspartnern zu einer Veränderung eines Fluoreszenzsignals eines Fluoreszenzreporters führt, wobei der Fluoreszenzreporter ein potential-sensitives Färbemittel, ein ionensensitives Färbemittel oder ein pH-sensitives Färbemittel ist,
wobei
- mindestens ein Interaktionspartner ein biochemischer Rezeptor, ein Ionenkanal oder eine Ionenpore ist, und
- mindestens ein Interaktionspartner sich in oder an einer Zelle, einem Vesikel oder organischem Gewebe befindet.

2. Verfahren nach Anspruch 1, bei dem die zeitliche Abfolge der Messwerte eine Zeitphase ansteigender Messwerte und/oder eine Zeitphase abnehmender Messwerte aufweist.

3. Verfahren nach Anspruch 1 oder 2, bei dem der mindestens eine erste Parameter eine Anstiegszeit oder eine Anstiegsrate kennzeichnet, welche der Zeitphase ansteigender Messwerte entspricht, und wobei der mindestens eine zweite Parameter eine Abklingzeit oder Abklingrate kennzeichnet, welche der Zeitphase abnehmender Messwerte entspricht.

4. Verfahren nach einem der Ansprüche 1-3, bei dem das mathematische Modell separate Funktionen verwendet, um Phasen der ansteigenden und der abnehmenden Messwerte der Abfolge der Messungen zu beschreiben.

5. Verfahren nach einem der Ansprüche 1-4, bei welchem zwei mathematische Modelle verwendet werden, um die Phase der abnehmenden Messwerte der Abfolge der Messungen zu beschreiben.

6. Verfahren nach einem der Ansprüche 1-5, bei welchem das mathematische Modell Abschnitte einzelner Gauss'scher Funktionen oder eine Überlagerung von Abschnitten mehrerer Gauss'scher Funktionen verwendet, um die Phase der ansteigenden Messwerte und die Phase der Abnehmenden Messwerte zu beschreiben.

7. Verfahren nach einem der Ansprüche 1-6, bei dem das mathematische Modell durch die numerische Least-Squares-Fit-Technik angepasst wird.

8. Verfahren nach einem der Ansprüche 1-7, bei dem mindestens ein Interaktionspartner ein G-Protein-gekoppelter Rezeptor ist.

9. Verfahren nach einem der Ansprüche 1-8, bei dem mindestens ein Interaktionspartner in einer Flüssigkeit gelöst oder suspendiert ist.

10. Verfahren nach einem der Ansprüche 1-9, bei dem die Interaktion des ersten Interaktionspartners mit mehreren zweiten Interaktionspartnern in mehreren Experimenten untersucht wird, wobei jedes Experiment die folgenden Schritte aufweist:
- Bringen der mindestens zwei Interaktionspartner in Kontakt miteinander,
- Durchführen einer zeitlichen Abfolge von Messungen, die jeweils einen Messwert erzeugen, welcher den Zustand der Interaktion zu einem bestimmten Zeitpunkt beschreibt,
- Wählen eines mathematischen Modells zur Beschreibung der zeitlichen Abfolge von Messungen, wobei das Modell mindestens einen ersten Parameter, welcher eine Zeitphase ansteigender Messwerte kennzeichnet, und mindestens einen zweiten Parameter aufweist, welcher eine Zeitphase abnehmender Messwerte kennzeichnet,
- Anpassen des mathematischen Modells an die zeitliche Abfolge von Messungen, wobei Werte für die Parameter bestimmt werden, welche zu einer guten Annäherung an die zeitliche Abfolge der Messungen durch das mathematische Modell führen, und
- anschließendes Verwenden einer statistischen Analyse der resultierenden Vielzahl von Werten der Parameter und/oder der Messungen der Abweichung des mathematischen Modells von der zeitlichen Abfolge der Messungen bei der Erkennung der Vielzahl von biochemischen Interaktionen.

11. Verfahren nach einem der Ansprüche 1 -10, bei dem der Startpunkt einer zeitlichen Entwicklung der biochemischen Interaktion durch das Bringen der Interaktionspartner in Kontakt miteinander definiert wird.

12. Verfahren nach einem der Ansprüche 1-11, bei dem der Startpunkt der zeitlichen Entwicklung der biochemischen Interaktion durch ein erstes externes Auslöseereignis definiert wird, nachdem die Interaktionspartner in Kontakt miteinander gebracht wurden.

13. Verfahren nach einem der Ansprüche 1-12, bei dem eine Veränderung der Richtung der zeitlichen Entwicklung der Messwerte durch ein zweites externes Auslöseereignis definiert wird, wobei die Veränderung einen Übergang von einer Phase ansteigender zu einer Phase abnehmender Messwerte, oder umgekehrt, umfasst.

14. Verfahren nach einem der Ansprüche 1-13, bei dem Lumineszenzsignale, vorzugsweise Fluoreszenzsignale, gemessen werden, um Messwerte zu erzeugen, welche den Zustand der Interaktion beschreiben.

15. Verfahren nach einem der Ansprüche 1-14, bei dem der Schritt des Erkennens der biochemische Interaktion durch Auswerten der Werte des ersten und des zweiten Parameters und/oder der Messung der Abweichung des mathematischen Modells von der zeitlichen Abfolge der Messungen Informationen über die Spezifität der Interaktion liefert.

16. Verfahren nach einem der Ansprüche 1-15, bei dem der Schritt des Erkennens der biochemischen Interaktion durch Auswerten der Werte des ersten und des zweiten Parameters Informationen über die Auswirkungen von Messartefakten auf die Messwerte liefert.

17. Verfahren nach einem der Ansprüche 1-16, bei dem das Bestimmen des Maßes der Abweichung des mathematischen Modells von der zeitlichen Abfolge der Messungen die folgenden Schritte aufweist:
- bei einer Vielzahl von aus der zeitlichen Abfolge der Messungen gewählten Messwerten, Berechnen der Differenz zwischen jedem Messwert und dem entsprechenden Wert des mathematischen Modells, wobei der mindestens eine erste und zweite Parameter, der durch Anpassen des Modells an die Abfolge der Messungen bestimmt wird, in dem Modell verwendet wird,
- Berechnen der Quadtrate dieser Differenzen,
- Berechnen einer gewichteten Summe der Quadrate, wobei die Gewichtung jedes Quadrats vorzugsweise von dem entsprechenden Messwert oder dem Wert des mathematischen Modells abhängt.

## Revendications

1. Procédé de détection d'une interaction biochimique entre au moins deux agents d'interaction, le procédé comprenant les étapes suivantes:
- mettre en contacte l'un avec l'autre des au moins deux agents d'interaction,
- établir une séquence chronologique de mesures, chaque mesure produisant une valeur mesurée décrivant l'état de l'interaction à un moment défini,
- choisir un modèle mathématique pour décrire la séquence chronologique de mesures, ledit modèle comprenant au moins un premier paramètre caractérisant une phase temporelle de valeurs mesurées croissantes et au moins un deuxième paramètre caractérisant une phase temporelle de valeurs mesurées décroissantes,
- adapter le modèle mathématique à la séquence chronologique de mesures, ainsi déterminant des valeurs pour lesdits paramètres qui donnent une bonne approximation de la séquence chronologique de mesures par le modèle mathématique, et
- détecter l'interaction biochimique par évaluation des valeurs du premier et du deuxième paramètre et/ou une mesure de la déviation du modèle mathématique de la séquence chronologique de mesures, l'interaction entre les au moins deux agents d'interaction résultant en un changement d'un signal fluorescent venant d'un rapporteur de fluorescence, ledit rapporteur de fluorescence étant un colorant sensible au potentiel, un colorant sensible aux ions ou un colorant sensible au pH,
dans lequel
- au moins un agent d'interaction est un récepteur biochimique, un canal ionique ou un pore ionique, et
- au moins un agent d'interaction est situé dans ou sur une cellule, une vésicule ou de tissu organique.

2. Procédé selon la revendication 1, dans lequel la séquence chronologique de valeurs de mesure comprend une phase temporelle de valeurs mesurées croissantes et/ou une phase temporelle de valeurs mesurées décroissantes.

3. Procédé selon la revendication 1 ou 2, dans lequel ledit au moins un premier paramètre caractérise un temps de croissance ou une vitesse de croissance correspondant à la phase temporelle de valeurs mesurées croissantes, et dans lequel ledit au moins un paramètre caractérise un temps de décroissance ou une vitesse de décroissance correspondant à la phase temporelle de valeurs mesurées décroissantes.

4. Procédé selon l'une quelconque des revendications 1-3, dans lequel le modèle mathématique utilise des fonctions séparées pour décrire les phases valeur mesurées croissantes et décroissantes de ladite séquence de mesures.

5. Procédé selon l'une quelconque des revendications 1-4, dans lequel deux modèles mathématiques sont utilisés pour décrire la phase de valeurs mesurées décroissantes de la séquence de mesures.

6. Procédé selon l'une quelconque des revendications 1-5, dans lequel le modèle mathématique utilise sections de fonctions Gaussiennes individuelles ou une superposition de section de plusieurs fonctions Gaussiennes afin de décrire la phase de valeurs mesurées croissantes et la phase de valeurs mesurées décroissantes.

7. Procédé selon l'une quelconque des revendications 1-6, dans lequel le modèle mathématique est adapté par moyen d'une ajustement numérique aux moindre carrés.

8. Procédé selon l'une quelconque des revendications 1-7, dans lequel au moins un agent d'interaction est un récepteur couplé aux protéines G.

9. Procédé selon l'une quelconque des revendications 1-8, dans lequel au moins un agent d'interaction est dissolu ou suspendu dans un liquide.

10. Procédé selon l'une quelconque des revendications 1-9, dans lequel l'interaction d'un premier agent d'interaction avec plusieurs deuxième agents d'interaction est examiné dans plusieurs essais, chaque essai comprenant les étapes suivantes:
- mettre en contacte l'un avec l'autre des au moins deux agents d'interaction,
- établir une séquence chronologique de mesures, chaque mesure produisant une valeur mesurée décrivant l'état de l'interaction à un moment défini,
- choisir un modèle mathématique pour décrire la séquence chronologique de mesures, ledit modèle comprenant au moins un premier paramètre caractérisant une phase temporelle de valeurs mesurées croissantes et au moins un deuxième paramètre caractérisant une phase temporelle de valeurs mesurées décroissantes,
- adapter le modèle mathématique à la séquence chronologique de mesures, ainsi déterminant des valeurs pour lesdits paramètres qui donnent une bonne approximation de la séquence chronologique de mesures par le modèle mathématique, et
- après, utiliser une analyse statistique de la pluralité résultante de valeurs des paramètres et/ou des mesures de la déviation du modèle mathématique de la séquence chronologiques de mesures, en détectant la pluralité d'interactions biochimiques.

11. Procédé selon l'une quelconque des revendications 1-10, dans lequel un moment de départ d'une évolution temporelle de l'interaction biochimique est défini par la mise en contacte des agents d'interaction.

12. Procédé selon l'une quelconque des revendications 1-11, dans lequel un moment de départ de l'évolution temporelle de l'interaction biochimique est défini par un premier événement déclencheur externe, après la mise en contacte des agents d'interaction.

13. Procédé selon l'une quelconque des revendication 1-12, dans lequel un changement de direction de l'évolution temporelle de valeurs mesurées est défini par un deuxième événement déclencheur externe, le changement comprenant une transition d'une phase de valeurs mesurées croissantes à une phase de valeurs mesurées décroissantes ou inverse.

14. Procédé selon l'une quelconque des revendication 1-13, dans lequel des signaux de luminescence, de préférence des signaux fluorescents, sont mesurés afin de produire des valeurs mesurées décrivant l'état de l'interaction.

15. Procédé selon l'une quelconque des revendication 1-14, dans lequel l'étape de détection de l'interaction biochimique par évaluation es valeurs du premier et du deuxième paramètre et/ou des mesures de la déviation du modèle mathématique de la séquence chronologique de mesures donne des informations sur la spécifité de l'interaction.

16. Procédé selon l'une quelconque des revendication 1-15, dans lequel l'étape de détection de l'interaction biochimique par évaluation du premier et du deuxième paramètre donne des informations sur l'effet des artefacts de mesure sur les valeurs mesurées.

17. Procédé selon l'une quelconque des revendication 1-16, dans lequel la détermination le mesure de déviation du modèle mathématique de la séquence chronologique de mesures comprend les étapes suivantes:
- pour plusieurs valeurs mesurées choisies dans la séquence chronologique de mesures, calculer la différence entre chaque valeur mesurée et la valeur correspondante du modèle mathématique, ledit au moins un premier paramètre et ledit au moins un deuxième paramètre déterminés par adaptation du modèle à la séquence de mesures étant utilisés dans ledit modèle,
- calculer les carrés de ladite différence,
- calculer une somme pondérée desdites carrés, le poids de chaque carré préférentiellement dépendant de la valeur mesurée correspondante ou la valeur du modèle mathématique.
